# FASCICULE DE BREVET EUROPEEN

(11) **EP 0 433 102 B1**
(45) Date de publication et mention de la délivrance du brevet: **20.09.1995**
(21) Numéro de dépôt: 90403068.1
(22) Date de dépôt: 30.10.1990
(51) Int. Cl.: A61F 5/448

(54) **Accouplement d'ostomie**
Ostomiekupplung
Ostomy coupling

(30) Priorité: 13.11.1989 FR 8914834
(43) Date de publication de la demande: 19.06.1991
(73) Titulaire: B. BRAUN BIOTROL, 92100 Boulogne Billancourt (FR)
(72) Inventeur: Holtermann, Henri, F-64500 Saint-Jean de Luz (FR)
(74) Mandataire: Ores, Irène

(56) Documents cités:
- DE-C- 284 947
- FR-A- 2 626 464
- GB-A- 2 217 207
- US-A- 1 689 085
- US-A- 3 964 485

## Description

La présente invention a pour objet un appareillage pour stomie.

On sait, comme excellemment explicité dans l'ouvrage de H. BAUMEL et J.F. LOUIS "Vivre avec une stomie digestive ou urinaire" (Simep, Villeurbanne, Paris, 1986) que : "La perte de l'incontinence intestinale ou urinaire consécutive à la création d'une stomie ne peut être compensée que par le port d'un appareillage destiné à recueillir les matières ou les urines". De nombreux appareillages ont par conséquent été proposés de longue date pour ce recueil et l'ouvrage cité en distingue trois grandes variétés, aussi bien pour les entérostomies (iléostomies, colostomies,...) que pour les urostomies, à savoir des dispositifs constitués par des poches à usage unique fixées directement à la peau de l'utilisateur par un adhésif en forme d'anneau, ou de telles poches munies d'un protecteur cutané, ou encore des systèmes "deux-pièces" dans lesquels la poche de recueil, -sous forme d'un sac jetable ou vidangeable-, peut être solidarisée de manière amovible sur une plaque frontale protectrice fixée au corps de l'utilisateur par un patin adhésif ou par une ceinture.

Des systèmes du type de ceux mentionnés en dernier lieu doivent satisfaire à des conditions impératives dont certaines sont contradictoires entre elles. Il convient, en effet, que la fixation de la poche de recueil sur la plaque frontale protectrice ou porte-poches puisse se faire sans application d'une pression importante dans la zone voisine de la stomie, -qui est sensible et généralement douloureuse-, et que la fixation soit sûre avec une parfaite étanchéité de l'assemblage du système tout en permettant une séparation aisée mais jamais intempestive de la poche. Complémentairement, l'appareillage ne doit pas être à l'origine de complications cutanées péristomiales, doit être aussi discret que possible et pouvoir s'adapter à des patients de morphologies très différentes. Il convient en outre, qu'après apprentissage par le personnel soignant, les patients puissent procéder eux-mêmes à la mise en place des poches de recueil sur le porte-poches, ce qui implique, bien entendu, et en particulier pour des utilisateurs d'un certain âge, que les manipulations d'assemblage et de séparation de la poche et du porte-poches soient aussi simples et aisées que possible.

Aussi, pour tenter d'apporter une solution au problème posé, a-t-on récemment proposé, par exemple dans FR-A1-2 626 464, un système "deux-pièces" selon le préambule de la revendication 1. Dans ce dernier, les moyens d'accouplement de la poche et du porte-poches sont constitués par un collet déformable et une bague fendue agencée pour serrer le pourtour de ce collet, une tubulure ayant une dimension extérieure sensiblement égale à la dimension intérieure du collet pouvant être insérée ou retirée en coulissant dans le collet quand la bague est dans une position d'ouverture, de sorte que, quand la bague est en position serrée autour du collet, celui-ci est déformé autour de la tubulure. L'actionnement de cette bague fendue est relativement malaisé, d'une part, et la réalisation de l'ensemble relativement complexe, d'autre part, étant donné que les diamètres de la tubulure et du collet doivent être réalisés avec de très faibles tolérances dimensionnelles pour que l'assemblage à étanchéité soit obtenu par une déformation réduite du collet dont la section U fait normalement obstacle à cette déformation.

On comprend alors que, nonobstant les très nombreux systèmes proposés du type à emboîtement, comme décrit par exemple dans EP-A-0 171 255, ou du type de celui décrit ci-avant, ou encore du type de celui selon GB-A-2 217 207 dans lequel une bague de serrage coopère avec des embouts de formes conjuguées de la poche et du porte-poches, le problème existe encore de fournir un appareillage pour stomie perfectionné qui permette de remplir les différentes exigences requises pour ces appareillages.

C'est, par conséquent, un but de l'invention de fournir un tel appareillage applicable aux stomies digestives ou urinaires qui satisfasse ces conditions et qui, en outre, soit suffisamment simple pour ne pas être d'un coût prohibitif.

C'est, aussi, un but de l'invention de fournir un tel appareillage qui permette de modifier facilement et sans risque pour le patient la position relative de la poche de recueil et du porte-poches.

C'est, également, un but de l'invention de fournir un tel appareillage dans lequel la mise en place de la poche sur la plaque frontale ou porte-poches préalablement fixée au corps d'un utilisateur soit extrêmement aisée, d'une part, et dans lequel, d'autre part, une légère inexactitude de présentation de la poche sur le porte-poches soit automatiquement corrigée tandis qu'une plus grande inexactitude interdit une fixation défectueuse et les conséquences dommageables qui peuvent en résulter pour le patient.

La présente invention apporte une solution au problème posé en proposant un appareillage pour stomie, comportant une plaque frontale ou porte-poches destiné à être fixé autour d'une ouverture artificielle du corps d'un utilisateur à l'aide d'un patin adhésif sensible à la pression, une ceinture ou analogue, une poche de recueil des fluides et/ou déchets corporels propre à être assemblée de manière amovible au porte-poches par un nez qu'elle présente et qui est prévu pour coopérer avec un embout solidaire de la plaque et qui entoure la stomie dans sa condition d'utilisation, **l'assemblage du nez de poche et de l'embout résultant de l'effet de serrage d'un organe déformale**, caractérisé en ce que :
- ledit appareillage comporte un mécanisme de commande associé au porte-poches et distinct de l'organe déformable, ce dernier également associé au porte-poches ;
- le nez de poche est logé, en condition d'assemblage, entre l'embout et l'organe déformable à l'intérieur de moyens de maintien portés par le porte-poches et constitués par l'embout qui est érigé sur une embase en forme de collerette et est entouré à distance par une paroi cylindrique qui ménage avec l'embout un espace prévu pour le logement dudit organe, de sorte que ce dernier est propre à coopérer directement avec le nez de poche, étant de forme et de dimension telles qu'il produit une liaison mécanique étanche entre ledit nez et l'embout en réponse à un actionnement dudit mécanisme.

Dans une forme de réalisation préférée, la paroi cylindrique est une paroi à retours dirigée sensiblement parallèlement à ladite collerette.

Avantageusement, l'organe de serrage est rendu opératoire lorsque le mécanisme approprié est actionné après qu'une poche ait été mise en place sur le porte-poches avec son nez logé entre ledit embout et ledit organe.

De manière préférée, l'organe de serrage est sous forme d'une partie d'anneau et en ce que le mécanisme associé au porte-poches est organisé pour rapprocher l'une des extrémités dudit organe de l'autre extrémité dont elle était initialement distante.

Dans une première forme de réalisation, le mécanisme d'actionnement de l'organe de serrage pour rapprocher l'une de l'autre les extrémités initialement distantes dudit organe est du type à genouillère.

Dans une autre forme de réalisation, le mécanisme d'actionnement de l'organe de serrage est du type à excentrique ou analogue.

Dans une autre forme de réalisation, la déformation de l'organe de serrage en réponse a l'actionnement du mécanisme est obtenue par engrènement de dents à une extrémité de l'organe de serrage et de dents de formes conjuguées prévues sur un levier monté à pivotement sur ladite pièce.

Avantageusement, ledit levier est monté à pivotement autour d'un axe matérialisé par deux tétons qu'il porte et qui sont engagés dans des oeils de formes conjuguées de deux joues en saillie radiale sur ladite pièce. Il s'ensuit une grande facilité d'actionnement par l'utilisateur.

Pour garantir le maintien sûr de la poche sur le porte-poches aussi longtemps que la poche ne doit pas être changée, les moyens de verrou sont associés au mécanisme assurant le rapprochement des deux parties d'extrémité de l'organe de serrage.

Quel que soit le type de réalisation, l'embout est plus rigide que le nez de poche de sorte que la surface intérieure du nez de poche coopère avec la surface extérieure de l'embout avec création d'une zone de contact linéaire propre à assurer l'étanchéité de l'appareillage sous l'action de l'organe de serrage qui agit par un effet de formes conjuguées.

Dans une forme de réalisation avantageuse, le nez de poche est en un matériau souple élastiquement déformable et a une section droite qui est conjuguée de celle de l'organe de serrage, d'une part, et est appariée à celle de l'embout du porte-poches, d'autre part, pour fournir la liaison étanche de la poche et du porte-poches.

L'étanchéité de la jonction du nez de poche et du porte-poches est réalisée par au moins un contact de type linéaire entre la surface externe de l'embout du porte-poches et la surface interne du nez de poche.

Dans une forme de réalisation préférée, la surface de l'embout avec laquelle coopère le nez de poche est tronconique et celle dudit nez de poche bombée ou vice-versa.

A la sécurité de fonctionnement de l'appareillage contribue également le fait que la section droite du nez de poche et celle de l'organe de serrage sont conjuguées de manière telle que l'actionnement du mécanisme assurant le rapprochement des extrémités de l'organe de serrage est impossible aussi longtemps que le nez de poche n'est pas correctement positionné par rapport audit organe de serrage et à l'embout du porte-poches tandis que pour un positionnement quasi-satisfaisant, le début de l'actionnement dudit mécanisme entraîne, par un effet de guidage, la mise en place parfaite des parties constitutives de l'appareillage les unes par rapport aux autres jusqu'au blocage, par effet de formes conjuguées, desdites parties entre elles.

Le nez de poche est avantageusement façonné par moulage d'une matière plastique, de préférence à base d'un copolymère d'éthylène et d'acétate de vinyle (EVA) ou en un matériau polymère compressible, comme une mousse de polyuréthanne ou encore, et de façon préférée, en un matériau alvéolaire à peau surfacique intégrée, à base de polyéthylène ou d'un copolymère d'éthylène et d'acétate de vinyle ou de polyuréthanne ou d'un matériau analogue tandis que l'organe de serrage est avantageusement en une matière plastique rigide façonnée par moulage dans sa condition "ouverte" pour reprendre cette condition après serrage et est, de préférence, en polypropylène ou en polyamide, ou en copolymère ABS ou en polyéthylène haute densité.

De manière préférée, le nez de poche est à base d'un copolymère d'EVA ou d'un élastomère de polyuréthanne ou encore, et de façon préférée, en un matériau alvéolaire à peau surfacique intégrée à base de copolymère d'EVA ou d'un élastomère de polyuréthanne ou d'un matériau analogue.

Dans une forme de réalisation avantageuse de l'appareillage, l'embout est conformé à son extrémité distante du patin adhésif et à l'intérieur de son ouverture suivant un rebord permettant le montage et le maintien d'un anneau ou coupelle de convexité particulièrement utile dans l'appareillage de certaines stomies invaginées.

L'invention sera bien comprise par la description qui suit, faite à titre d'exemple et en référence au dessin annexé, dans lequel :
- la Figure 1 est une vue schématique en perspective montrant, distant l'un de l'autre, une plaque frontale ou porte-poches et un nez de poche d'un appareillage selon l'invention ;
- la Figure 2 est une vue en élévation du porte-poches et d'un organe de serrage qu'il enferme ;
- la Figure 3 est une vue en coupe selon la ligne 3-3 de la Figure 2 ;
- la Figure 4 est une vue schématique en coupe d'une poche d'appareillage selon l'invention ;
- la Figure 5 est une vue partielle, à plus grande échelle, d'un appareillage selon l'invention dans la condition d'assemblage de la poche sur le porte-poches ;
- la figure 6 est une vue d'une partie constitutive du porte-poches ;
- la figure 7 est une vue en coupe selon la ligne 7-7 de la figure 6 ;
- la figure 8 est une vue en élévation d'un organe de serrage d'un dispositif selon l'invention ;
- la figure 9 est une vue en élévation d'un levier d'actionnement ;
- la figure 10 est une vue en coupe selon la ligne 10-10 de la figure 9 ;
- la figure 11 est une vue en coupe selon la ligne 11-11 de la figure 9 ;
- la figure 12 illustre une variante de réalisation ;
- la figure 13 est une vue en coupe illustrant une autre forme de réalisation.

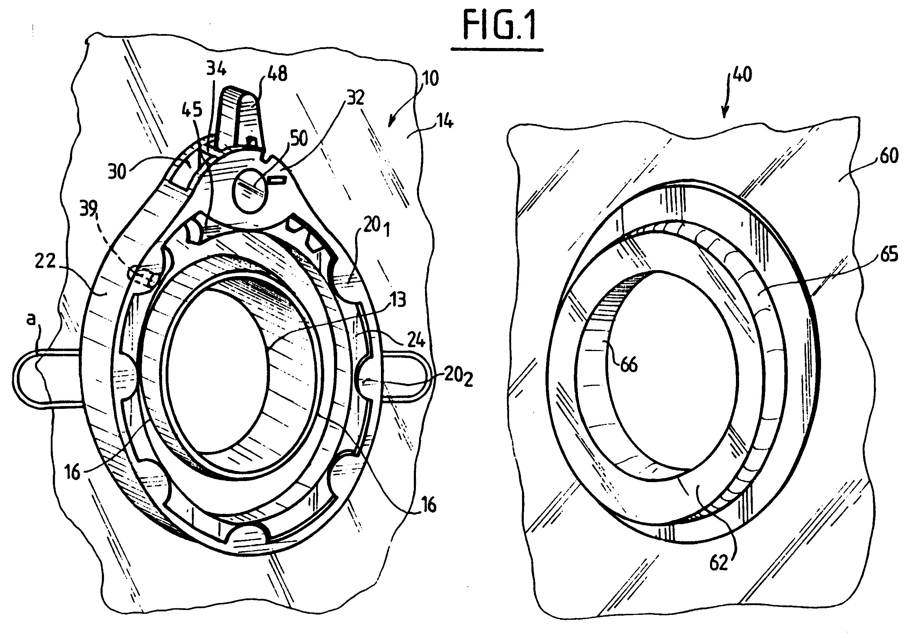

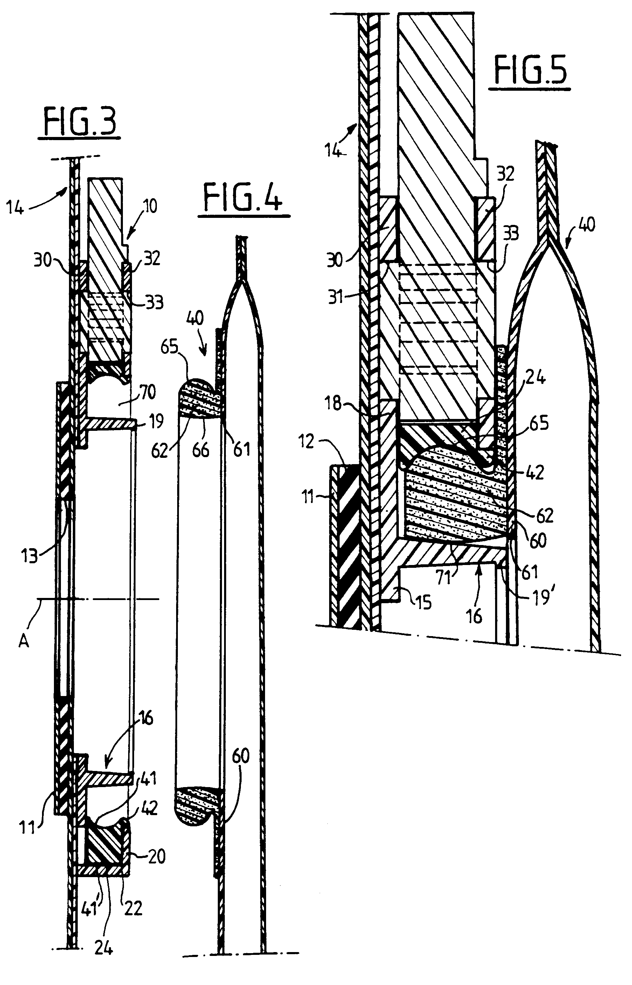

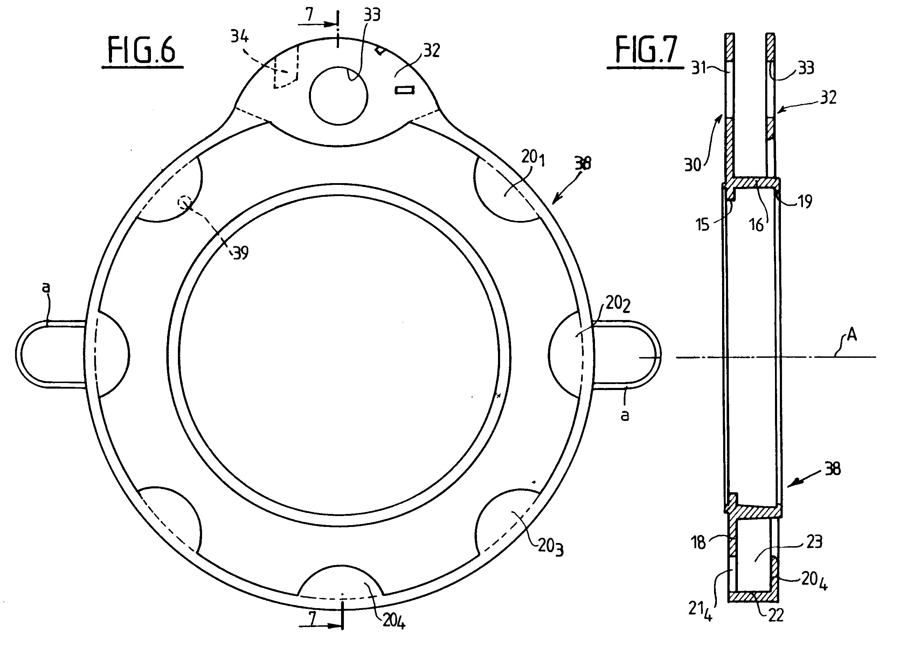

On se réfère d'abord aux figures 1 à 11. Elles montrent un appareillage pour stomie selon l'invention du type "deux-pièces", c'est-à-dire comprenant une plaque frontale ou porte-poches 10 prévu pour être fixé autour d'une ouverture artificielle du corps d'un utilisateur à l'aide d'un moyen de fixation approprié comme un patin adhésif sensible à la pression 12, en soi connu, et qui est protégé aussi longtemps que le dispositif n'est pas mis en oeuvre par une pellicule 11, figures 3 et 5, qui peut être retirée par pelage ; en variante et/ou complémentairement, le porte-poches 10 peut être maintenu sur le corps de l'utilisateur à l'aide d'une ceinture (non représentée) ou d'un moyen analogue propre à être fixé à l'aide de petites anses, a.

Le porte-poches 10, dont le patin adhésif 12 présente une ouverture 13 entourant la stomie dans sa condition d'utilisation comprend également, sur la face de l'adhésif 12 opposée à celle venant au contact du corps de l'utilisateur, une feuille composite 14 qui contribue à la protection de la zone péristomiale et au maintien sur la peau de l'utilisateur. Sur la face de la feuille composite 14 opposée à celle de liaison au patin 12 est fixé, par une bague interne 15 qu'il présente, un embout moulé en matière plastique 16. La fixation peut être par soudure, par exemple du type thermique, haute fréquence ou encore par collage avec ou sans interposition de film(s) compatible(s) avec les matériaux constitutifs de l'embout et de la feuille 14, lesquels peuvent être réalisés en polyéthylène, haute ou basse densité, ou en copolymère d'éthylène et d'acétate de vinyle (EVA) ou en polychlorure de vinyle (PVC) ou en polyamide ou un mélange de ces matériaux, le film de la feuille composite 14 adjacent à la bague 15 étant avantageusement un film non tissé à base d'un ou de ces matériaux.

L'embout 16 est érigé à partir d'une embase 18 ayant une forme générale de collerette circulaire, sensiblement coplanaire à la bague 15 et est conformé suivant une surface tronconique s'inclinant progressivement sur l'axe A du dispositif, avec sur son bord libre 19 un rebord 19′ permettant le montage et le maintien d'un anneau ou coupelle de convexité, non représenté, particulièrement utile dans l'appareillage de certaines stomies invaginées. Comme bien visible sur les figures 1, 6 et 7, l'embout 16 à contour circulaire est entouré à distance par une paroi cylindrique 22 qui ménage avec l'embout un espace 23, le bord libre de la paroi 22 n'étant pas à contour circulaire (en regard de l'axe A) mais découpé suivant des lobes 20₁, 20₂, 20₃, etc... régulièrement répartis autour de l'axe A, en face de trous de même disposition, 21, ménagés dans l'embase 18.

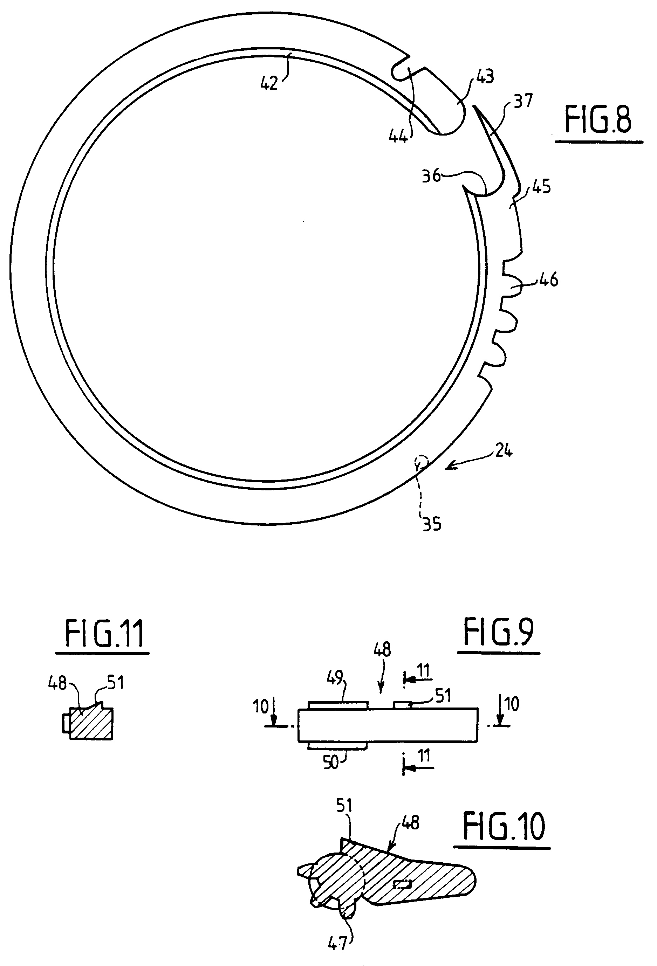

Conformément à l'invention, l'espace 23 est prévu pour le logement d'un organe de serrage 24, figure 8, qui est avantageusement moulé en matière plastique dans la forme circulaire et la condition "ouverte"' qui est celle montrée sur le dessin de la figure 8 de sorte qu'il en résulte un effet élastique de ressort quand ses extrémités qui ont été préalablement rapprochées sont libérées à l'écartement l'une de l'autre, comme il sera précisé ci-après.

Comme bien montré également sur les figures 1, 2, 6 et 7, l'embase 18 présente une première oreille radiale 30 percée d'un orifice 31 et, en regard de celle-ci et sensiblement dans le plan du rebord 19′ et des lobes 20, est prévue une seconde oreille 32 percée d'un orifice 33, les oreilles 30 et 32 étant reliées par un étroit pontet 34.

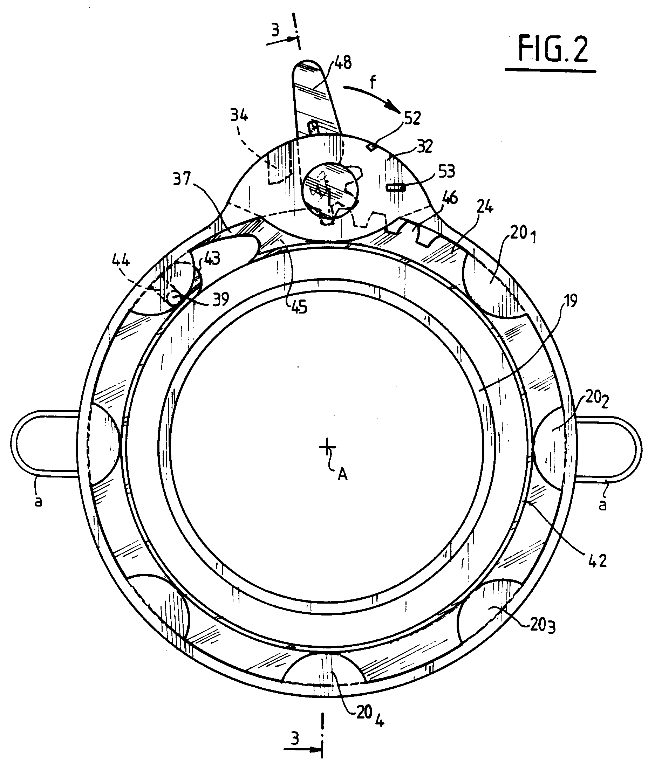

Comme montré sur les figures 2, 5 et 8, l'organe 24 (avantageusement réalisé en polypropylène, polyamide, copolymère ABS ou en polyéthylène haute densité) est constitué par une portion d'anneau en matière plastique élastiquement déformable, et il présente une section droite à surface interne 41 concave et surface externe 41′ cylindrique avec un cordon 42 pouvant venir au contact des parties les plus proches de l'axe A des lobes 20. Au voisinage d'une de ses extrémités, 43, l'organe 24 présente une boutonnière 44 propre à coopérer avec un pion 39 érigé sur l'embase 18 pour le maintien en position de cette extrémité 43, tandis que l'autre extrémité 45 de l'organe 24 est conformée suivant un bec 37 et un évidement concave 36 qui, lorsque les extrémités 43 et 45 sont rapprochées l'une de l'autre, donnent à l'organe 24 une forme très sensiblement circulaire. Un téton 35 sur la face de l'organe 24 opposée à celle présentant le cordon 42 est logé dans un des trous 21, pour le positionnement périphérique de l'organe 24 lequel présente également, au voisinage de son extrémité 45 et sur sa surface externe, des dents 46 avec lesquelles sont propres à engrener les dents de forme et pas conjugués, 47, d'un levier 48 monté à pivotement par ses tourillons 49 et 50, figure 9, dans les orifices 31 et 33 des oreilles 30 et 32, respectivement. Pour le maintien de l'effet de serrage introduit par l'organe 24 lorsque le levier 48 est actionné dans le sens de la flèche f, figure 2, faisant se rapprocher ainsi les extrémités 43 et 45, on prévoit dans une forme de réalisation qu'une des faces du levier 48 soit munie d'un ergot 51, propre à coopérer avec des crans conjugués 52 et 53 prévus sur une des oreilles, par exemple l'oreille 32, de la pièce 38 que forme l'ensemble monobloc de l'embout 16, l'embase 18, la paroi cylindrique 22, les lobes 20, la bague 15 et lesdites oreilles et le cas échéant, les anses a.

Dans une variante, un ergot 51′ est formé sur une face frontale du levier et le maintien de l'effet de serrage est obtenu par blocage dudit ergot au contact du pontet 34, après franchissement de ce dernier.

L'appareillage pour stomie selon l'invention comprend également une poche 40 du type à jeter ou à vider pour le recueil des fluides et/ou déchets corporels propres à être évacués au travers de l'embout 16. Elle peut être réalisée en un film de polyéthylène ou de PVC, ou de polyamide, ou en un film barrière complexe de type polyéthylène/EVA/polychlorure de vinyle/EVA/polyéthylène, comme ceux connus sous la Marque Déposée SARANEX de la Société DOW CHEMICAL ou un film complexe du type EVA/copolymère polychlorure de vinylidène-EVA, comme ceux connus sous la Marque CRYOVAC de la Société GRACE et sous la Marque Déposée SARANEX de la Société DOW CHEMICAL ou encore un film complexe du type EVA/EVOH/EVA, ou un matériau analogue.

Conformément à l'invention, à la poche 40 qui comprend une paroi 60 percée d'un trou 61, est associé un raccord ou nez de poche, 62, solidarisé de manière étanche avec la poche 40 sur laquelle il est soudé, ou collé, comme indiqué ci-dessus, de manière que son axe soit co-axial à celui du trou 61. Comme bien visible sur les figures 4 et 5, le raccord ou nez de poche 62 est conformé suivant une surface latérale externe 65, de même profil que la surface interne de l'organe 24 et une surface interne 66 incurvée ou bombée. Conformément à l'invention également, le nez de poche 62 en un matériau souple, avantageusement une matière plastique alvéolaire, de préférence à base d'un copolymère d'éthylène et d'acétate de vinyle (EVA) ou un matériau polymère compressible, comme une mousse de polyuréthanne ou encore, et de façon préférée, en un matériau alvéolaire à peau surfacique intégrée, par exemple de polyéthylène, ou d'un copolymère d'éthylène et d'acétate de vinyle, ou de polyuréthanne, de sorte que, et contrairement aux dispositifs connus, le nez de poche peut être déformé, voire plié pour faciliter sa mise en place sur la plaque frontale ou porte-poches 10.

Le fonctionnement d'un appareillage selon l'invention résulte immédiatement de ce qui précède.

Le porte-poches 10 (c'est-à-dire l'ensemble de la pièce 38, de ses moyens de fixation au corps de l'utilisateur, de l'organe 24 et de son mécanisme d'actionnement) étant fixé au corps de l'utilisateur, par exemple à l'aide d'une ceinture et des anses a et/ou par l'effet d'adhésion du patin 12 et de la feuille 14 (après qu'ait été retirée, bien entendu, la pellicule de protection 11), le levier 48 est dans la condition montrée sur la figure 2, en laquelle ses dents 47 coopèrent avec les dents 46 de l'organe 24 dont les extrémités 43 et 45 sont alors distantes l'une de l'autre. Dans cette condition, la poche 40 peut être présentée en regard du porte-poches et le nez de poche 62, souple, introduit sans effort et sans difficulté dans l'espace annulaire 70, figure 3, régnant entre la surface latérale externe de l'embout 16 et la surface latérale interne 41 de l'organe 24. Lorsque, à partir de cette condition, on fait pivoter le levier 48 dans le sens de la flèche f montré sur la figure 2, le diamètre de l'organe 24 est réduit, -les extrémités 43 et 45 se rapprochant l'une de l'autre-, et il en résulte un effort de serrage dudit organe sur le raccord ou nez de poche 62 qui provoque l'immobilisation ou le blocage par un effet de formes conjuguées de la poche sur le porte-poches.

Lorsqu'au cours du déplacement du levier 48 l'ergot 51 passe le premier cran 52 qu'il rencontre sur son chemin, le serrage est tel que la poche 40 peut encore être déplacée par rapport au porte-poches, non pas à l'extraction, cependant, mais par une rotation autour de son axe, par exemple pour modifier le positionnement de ladite poche par rapport au corps de l'utilisateur, ou faciliter une vidange, etc. Lorsque, par contre, le mouvement de pivotement du levier 48 se poursuit, jusqu'à ce que l'ergot 51 franchisse le cran 53, ou que, dans la variante également illustrée l'ergot 51′ franchisse le pontet 34, alors la force de serrage est telle qu'une séparation intempestive de la poche et du porte-poches est impossible.

Dans cette condition, également, la surface intérieure 66 du raccord 62 coopère avec la surface extérieure de l'embout 16 avec création d'une zone de contact linéaire, 71, figure 5, qui assure l'étanchéité entre ledit embout et la poche, lesquels sont en outre mécaniquement liés l'un à l'autre.

C'est un processus inverse qui est mis en oeuvre pour séparer la poche 40 du porte-poches 10, après rotation du levier pivotant 48 dans le sens inverse de celui montré par la flèche f et après que l'on ait fait échaper l'ergot 51 au cran 53, ou l'ergot 51′ au pontet 34 tirant parti, d'une part, de l'action des dents 46 et 47 qui coopèrent entre elles mais aussi, d'autre part, de la nature élastique du matériau constitutif de l'organe 24.

A la parfaite sécurité de l'appareillage contribue également le fait que, en raison des profils appariés du raccord ou nez de poche 62 et de l'organe 24, l'actionnement du levier 48 jusqu'à la position en laquelle son ergot 51 coopère avec le cran 53 ou son ergot 51′ avec le pontet 34 est impossible aussi longtemps que le raccord 62 est mal positionné dans l'espace 70, tandis que si le positionnement dudit raccord dans ledit espace n'est pas satisfaisant, mais est proche de cette condition, la surface 41 de l'organe 24 agit comme un poussoir sur le raccord pour le mettre en place de façon tout à fait satisfaisante, préalablement au verrouillage par serrage à l'aide du levier 48.

Le fonctionnement de l'appareillage est simple et sûr et un fonctionnement très satisfaisant a été obtenu pour un appareillage comprenant un embout 16 de 50 mm de diamètre, une paroi 22 d'environ 70 mm de diamètre, un nez de poche d'environ 58 mm de diamètre externe et 52 mm de diamètre interne, l'organe de serrage 24 ayant une largeur d'environ 5 mm, avec deux dents au diamètre primitif d'environ 66 mm avec lesquelles coopèrent les dents conjuguées du levier.

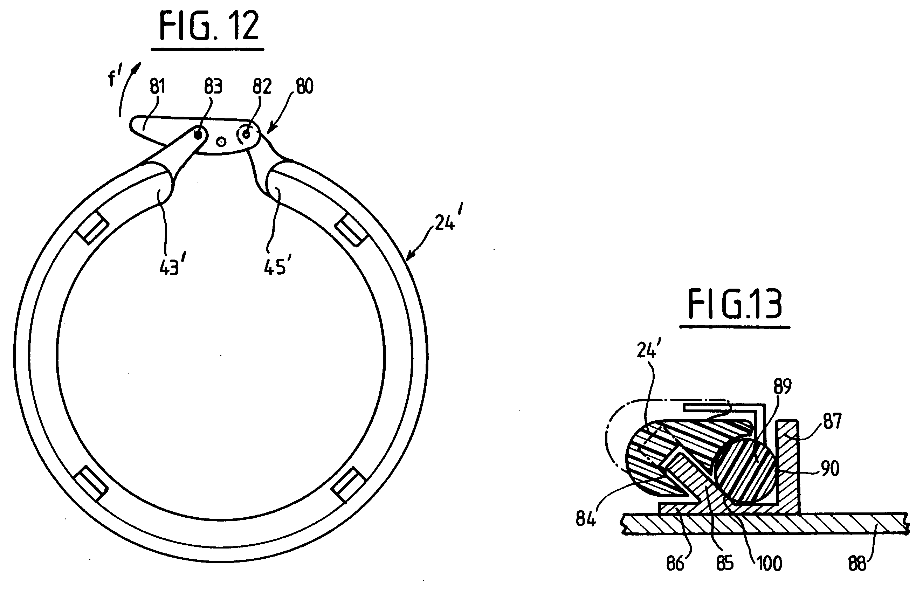

L'invention ne se limite nullement au mode de réalisation et d'application qui viennent d'être décrits de façon plus explicite. Elle en embrasse au contraire toutes les variantes qui peuvent venir à l'esprit du technicien en la matière. Ainsi, et comme montré sur la figure 12, l'organe 24′ analogue à l'organe 24 de la réalisation précédente, peut être muni à ses extrémités 43′ et 45′ (distantes l'une de l'autre aussi longtemps que la poche n'est pas montée sur le porte-poches), d'un mécanisme à genouillère 80, à levier 81 pivotant dans le sens de la flèche f′ autour d'un axe 82 pour entraîner l'extrémité 43′ articulée sur le levier autour d'un axe 83, au rapprochement de l'extrémité 45′.

Dans un tel appareillage où le porte-poches est montré schématiquement en 88, la section droite de l'organe 24′ peut être comme montrée sur la figure 13, c'est-à-dire à contour d'allure générale triangulaire avec une creusure 84 de guidage sur une nervure 85 en saillie sur l'embase 86 et s'étendant radialement à l'extérieur d'un embout 87 analogue à l'embout 16 de la réalisation précédente. Dans cette réalisation, le nez de poche 89, à section droite quelque peu circulaire, est mis en place sur le porte-poches lorsque l'organe 24′ est dans la condition montrée en trait mixte sur la figure 13 et, lorsque le mécanisme à genouillère 80 ou un mécanisme analogue est actionné, l'organe 24′ prend sa position montrée en trait plein sur cette figure pour serrer ledit nez de poche qui est immobilisé par effet de formes conjuguées entre la nervure 85 et la surface externe de l'embout 87 avec, dans une telle réalisation, deux zones linéaires d'étanchéité comme montré schématiquement en 90 et 100.

## Revendications

1. Appareillage pour stomie comportant une plaque frontale ou porte-poches (10) destiné à être fixé autour d'une ouverture artificielle du corps d'un utilisateur à l'aide d'un patin adhésif sensible à la pression (12), une ceinture ou analogue, une poche (40) de recueil des fluides et/ou déchets corporels propre à être assemblée de manière amovible au porte-poches par un nez (62) qu'elle présente et qui est prévu pour coopérer avec un embout (16) solidaire de la plaque et qui entoure la stomie dans sa condition d'utilisation, l'assemblage du nez de poche (62) et de l'embout (16) résultant de l'effet de serrage d'un organe déformable (24), caractérisé en ce que :
- ledit appareillage comporte un mécanisme de commande (48) associé au porte-poches et distinct de l'organe déformable (24), ce dernier également associé au porte-poches (10) ;
- le nez de poche (62) est logé, en condition d'assemblage, entre l'embout (16) et l'organe déformable (24) à l'intérieur de moyens de maintien portés par le porte-poches (10) et constitués par l'embout (16) qui est érigé sur une embase (18) en forme de collerette et est entouré à distance par une paroi cylindrique (22) qui ménage avec l'embout un espace (23) prévu pour le logement dudit organe (24), de sorte que ce dernier est propre à coopérer directement avec le nez de poche (62), étant de forme et de dimension telles qu'il produit une liaison mécanique étanche entre ledit nez et l'embout (16) en réponse à un actionnement dudit mécanisme (48).

2. Appareillage selon la revendication 1, caractérisé en ce que la paroi cylindrique (22) est une paroi à retours (20) dirigée sensiblement parallèlement à ladite collerette (18).

3. Appareillage selon la revendication 1 ou la revendication 2, caractérisé en ce que l'organe de serrage (24) est rendu opératoire lorsque le mécanisme approprié (48) est actionné après qu'une poche (40) ait été mise en place sur le porte-poches (10) avec son nez (62) logé entre ledit embout (16) et ledit organe (24).

4. Appareillage selon la revendication 3, caractérisé en ce que l'organe de serrage (24) est sous forme d'une partie d'anneau et en ce que le mécanisme associé au porte-poches (10) est organisé pour rapprocher l'une des extrémités (45, 45') dudit organe (24) de l'autre extrémité dont elle était initialement distante.

5. Appareillage selon la revendication 3 ou la revendication 4, caractérisé en ce que le mécanisme (80) d'actionnement de l'organe de serrage (24') pour rapprocher l'une de l'autre les extrémités initialement distantes dudit organe est du type à genouillère.

6. Appareillage selon la revendication 3 ou la revendication 4, caractérisé en ce que le mécanisme d'actionnement de l'organe de serrage est du type à excentrique ou analogue.

7. Appareillage selon la revendication 3 ou la revendication 4, caractérisé en ce que la déformation de l'organe de serrage (24) en réponse à l'actionnement du mécanisme (48) est obtenue par engrènement de dents (46) à une extrémité (45) de l'organe de serrage (24) et de dents (47) de formes conjuguées prévues sur un levier (48) monté à pivotement sur ladite pièce (38).

8. Appareillage selon la revendication 7, caractérisé en ce que ledit levier (48) est monté à pivotement autour d'un axe matérialisé par deux tétons (49, 50) qu'il porte et qui sont engagés dans des oeils (31, 33) de formes conjuguées de deux joues (30, 32) en saillie radiale sur ladite pièce (38).

9. Appareillage selon l'une quelconque des revendications 3 à 9, caractérisé en ce que le mécanisme d'actionnement comprend des moyens de verrou (51, 51' ; 52, 53, 34).

10. Appareillage selon l'une quelconque des revendications précédentes, caractérisé en ce que l'embout (16) est plus rigide que le nez de poche (62) de sorte que la surface intérieure du nez de poche coopère avec la surface extérieure de l'embout avec création d'une zone de contact linéaire propre à assurer l'étanchéité de l'appareillage sous l'action de l'organe (24) de serrage qui agit par un effet de formes conjuguées.

11. Appareillage selon l'une quelconque des revendications précédentes, caractérisé en ce que le nez de poche (62) est en un matériau souple élastiquement déformable et a une section droite qui est conjuguée de celle de l'organe de serrage (24), d'une part, et est appariée à celle de l'embout (16) du porte-poches (10), d'autre part, pour fournir la liaison étanche de la poche et du porte-poches.

12. Appareillage selon l'une quelconque des revendications précédentes, caractérisé en ce que l'étanchéité de la jonction du nez de poche (62) et du porte-poches (10) est réalisée par au moins un contact de type linéaire (71) entre la surface externe de l'embout (16) du porte-poches (10) et la surface interne (66) du nez de poche (62).

13. Appareillage selon l'une quelconque des revendications précédentes, caractérisé en ce que la surface de l'embout (16) avec laquelle coopère le nez de poche (62) est tronconique et celle (66) dudit nez de poche bombée ou vice-versa.

14. Appareillage selon l'une quelconque des revendications 3 à 13, caractérisé en ce que la section droite du nez de poche (62) et celle de l'organe de serrage (24) sont conjuguées de manière telle que l'actionnement du mécanisme (48) assurant le rapprochement des extrémités de l'organe de serrage (24) est impossible aussi longtemps que le nez de poche (62) n'est pas correctement positionné par rapport audit organe de serrage et à l'embout (16) du porte-poches (10) tandis que pour un positionnement quasi-satisfaisant, le début de l'actionnement dudit mécanisme entraîne, par un effet de guidage, la mise en place parfaite des parties constitutives de l'appareillage les unes par rapport aux autres jusqu'au blocage, par effet de formes conjuguées, desdites parties entre elles.

15. Appareillage selon l'une quelconque des revendications précédentes, caractérisé en ce que le nez de poche (62) est avantageusement façonné par moulage d'une matière plastique, de préférence à base d'un copolymère d'éthylène et d'acétate de vinyle (EVA) ou en un matériau polymère compressible, comme une mousse de polyuréthanne ou encore, et de façon préférée, en un matériau alvéolaire à peau surfacique intégrée, à base de polyéthylène ou d'un copolymère d'éthylène et d'acétate de vinyle ou de polyuréthanne ou d'un matériau analogue.

16. Appareillage selon l'une quelconque des revendications précédentes, caractérisé en ce que l'organe de serrage (24) est avantageusement en une matière plastique rigide façonnée par moulage dans sa condition "ouverte" pour reprendre cette condition après serrage et est, de préférence, en polypropylène ou en polyamide, ou en copolymère ABS ou en polyéthylène haute densité.

17. Appareillage selon la Revendication 15, caractérisé en ce que le nez (62) de poche est, de préférence, à base d'un copolymère d'EVA ou d'un élastomère de polyuréthanne ou encore, et de façon préférée, en un matériau alvéolaire à peau surfacique intégrée à base de copolymère d'EVA ou d'un élastomère de polyuréthanne ou d'un matériau analogue.

18. Appareillage selon l'une quelconque des revendications précédentes, caractérisé en ce que l'embout (16) est conformé à son extrémité distante du patin adhésif et à l'intérieur de son ouverture suivant un rebord (19') permettant le montage et le maintien d'un anneau ou coupelle de convexité particulièrement utile dans l'appareillage de certaines stomies invaginées.

## Claims

1. Stoma device comprising a frontal plate or a bag-carrier (10) intended to be fixed about an artificial opening in the body of a user with the aid of a pressure-sensitive adhesive pad (12), a belt, or similar, and a collecting bag (40) for body fluids and/or wastes, being suitable for assembly in a detachable manner with the bag-carrier by means of a nose (62) which is provided thereon and which is designed in order to cooperate with a joining piece (16) which is integral with the plate and which surrounds the stoma in its operational position, the assembly of the bag nose (62) and the joining piece (16) resulting from the clamping effect of a deformable member (24), characterised in that:
- said device comprises a control mechanism (48) associated with the bag-carrier and distinct from the deformable member (24), this latter also being associated with the bag-carrier (10);
- the bag nose (62) being lodged, in the assembled state, between the joining piece (16) and the deformable member (24) inside retaining means supported by the bag-carrier (10) and consisting of the joining piece (16) which is constructed on a seat (18) in the form of a flange and is surrounded at a distance by a cylindrical wall (22) which, together with the joining piece, provides a space (23) for the accommodation of said member (24) in such a manner that this latter is suitable for cooperating directly with the bag nose (62), being of a shape and measurements such that it produces a leaktight mechanical connection between said nose and the joining piece (16) in response to an activation of said mechanism (48).

2. Device according to Claim 1, characterised in that the cylindrical wall (22) is a return wall (20) directed substantially parallel to said flange (18).

3. Device according to Claim 1 or Claim 2, characterised in that the clamping member (24) is rendered operative when the appropriate mechanism (48) is actuated after a bag (40) has been positioned on the bag support (10) with the nose (62) thereof lodged between said joining piece (16) and said member (24).

4. Device according to Claim 3, characterised in that said clamping member (24) is in the form of a ring part and in that the mechanism associated with the bag carrier (10) is arranged to bring one end (45, 45') of the ends of said member (24) closer to the other end from which it is, initially, at a distance.

5. Device according to Claim 3 or Claim 4, characterised in that the mechanism (80) for actuating the clamping member (24') to bring one of the initially distant ends of said member closer to the other end thereof is of the toggle type.

6. Device according to Claim 3 or Claim 4, characterised in that the actuating mechanism of the clamping member is of the eccentric type or similar.

7. Device according to Claim 3 or Claim 4, characterised in that the deformation of the clamping member (24) in response to the activation of the mechanism (48) is obtained by meshing of teeth (46) at one end (45) of the clamping member (24) and teeth (47) of complementary shape provided on a lever (48) which is mounted in a pivoting manner on said part (38).

8. Device according to Claim 7, characterised in that said lever (48) is mounted in a pivoting manner about a shaft which is formed at two dog points (49, 50) which it supports and which are engaged in eyes (31, 33) of complementary shape of two projecting sheets (30, 32) which project radially on said part (38).

9. Device according to any one of Claims 3 to 9, characterised in that the actuating mechanism comprises locking means (51, 51'; 52, 53, 54).

10. Device according to any one of the preceding claims, characterised in that the joining piece (16) is more rigid than the bag nose (62) such that the inner surface of the bag nose cooperates with the outer surface of the joining piece to form a area of linear contact suitable for ensuring the leaktightness of the device under the action of the clamping member (24) which is made effective by means of complementary shapes.

11. Device according to any one of the preceding claims, characterised in that the bag nose (62) is of a resiliently deformable supple material and has a cross-section which is complementary to that of the clamping member (24), on the one hand, and is mated with that of the joining piece (16) of the bag carrier (10) on the other hand, to provide a leaktight connection of the bag and the bag carrier.

12. Device according to any one of the preceding claims, characterised in that the leaktightness of the connection of the bag nose (62) and the bag carriers (10) is performed by at least one contact (71) of the linear type between the external surface of the junction piece (16) of the bag carrier (10) and the internal surface (66) of the bag nose (62).

13. Device according to any one of the preceding claims, characterised in that the surface of the joining piece (16), with which the bag nose (62) cooperates is frustoconical and that (66) of said bag nose is bulging or vice versa.

14. Device according to any one of Claims 3 to 13, characterised in that the cross-section of the bag nose (62) and that of the clamping member (24) are complementary in a manner such that the activation of the mechanism (48) which brings the ends of the clamping member (24) closer together, is impossible as long as the bag nose (62) is not positioned correctly in relation to said clamping member and the joining piece (16) of the bag carrier (10), whereas, if positioning is particularly satisfactory, the beginning of the activation of said mechanism causes, by means of a guiding effect, the perfect positioning of the constituent parts of the device in relation to one another until they lock owing to the effect of the complementary shapes of said parties in relation to one another.

15. Device according to any one of the preceding claims, characterised in that the bag nose (62) is advantageously produced by moulding a plastics material, which is preferably based on an ethylene copolymer and vinyl acetate (EVA) or on a compressible polymer material such as a polyurethane foam, or, even in a preferred manner, of an alveolar material with an integrated surface skin, based on polyethylene or on an ethylene copolymer and vinyl acetate or on polyurethane or on a similar material.

16. Device according to any one of the preceding claims, characterised in that the clamping member (24) is, advantageously, of a rigid plastics material formed, by moulding, into its "open" condition, so that it will resume this position after clamping and is, preferably, made of polypropylene or polyamide or of ABS copolymer or of high-density polyethylene.

17. Device according to Claim 15, characterised in that the bag nose (62) is, preferably, based on an EVA copolymer or a polyurethane elastomer or even, and in a preferred manner, on an alveolar material with a surface skin integrated with an EVA copolymer or an elastomer of polyurethane or a similar material.

18. Device according to any one of the preceding claims, characterised in that the joining piece (16) is formed, at the end thereof which is remote from the adhesive pad inside its aperture following a shoulder (19') enabling a ring or cup to be mounted and retained in position, this ring or cup having a convexity which is particularly useful in the case of a device for certain invaginated stomas.

## Patentansprüche

1. Einrichtung für Ostomie, eine Stirnplatte oder einen Beutelträger (10) umfassend, der dazu bestimmt ist, um eine künstliche Körperöffnung eines Benützers herum mit Hilfe eines druckempfindlichen Klebehaftschuhs (12), eines Gürtels oder dergleichen, befestigt zu werden, einem Sammelbeutel (40) für Fluide und/oder Körperabscheidungen, der geeignet ist, in lösbarer Weise mit dem Beutelträger über eine Nase, die er aufweist, zusammengefügt zu werden und die vorgesehen ist, um mit einem mit der Platte festen Ansatz (16) zusammenzuwirken und der die Ostomie in ihrem Benützungszustand umschließt, wobei das Zusammenfügen der Beutelnase (62) und des Ansatzes (16) aus dem Spanneffekt eines verformbaren Organs (24) herrührt, **dadurch gekennzeichnet**,
- daß die Einrichtung einen Betätigungsmechanismus (48), zugeordnet zum Beutelträger und unterschiedlich zum verformbaren Organ (24), umfaßt, wobei letzteres ebenfalls dem Beutelträger (10) zugeordnet ist;
- die Beutelnase (62) im zusammengefügten Zustand zwischen dem Ansatz (16) und dem verformbaren Organ (24) im Inneren von durch den Beutelträger (10) getragenen Haltemitteln gelagert ist, die durch den Ansatz (16), der auf einer Grundplatte (18) in Bundform aufragt und der unter Abstand von einer zylindrischen Wand (22) umschlossen ist, die mit dem Ansatz einen Raum (23) bildet, der für die Lagerung dieses Organs (24) vorgesehen ist, derart, daß letzteres geeignet ist, direkt mit der Nase der Tasche (62) zusammenzuwirken und von einer Form und Abmessung derart, daß sich eine mechanische dichte Verbindung zwischen der Nase und dem Ansatz (16) in Abhängigkeit von einer Betätigung dieses Mechanismus (48) bildet.

2. Einrichtung nach Anspruch 1, dadurch gekennzeichnet, daß die zylindrische Wandung (22) eine umgebogene Wandung (20) ist, die im wesentlichen parallel zu diesem Bund (18) gerichtet ist.

3. Einrichtung nach Anspruch 1 oder Anspruch 2, dadurch gekennzeichnet, daß das Spannorgan (24) betätigbar wird, wenn der geeignete Mechanismus (48) betätigt wird, nachdem ein Beutel (40) an seinen Ort auf dem Beutelträger (10) gebracht ist, wobei seine Nase (62) zwischen diesem Ansatz (16) und diesem Organ (24) gelagert ist.

4. Einrichtung nach Anspruch 3, dadurch gekennzeichnet, daß das Spannorgan (24) in Form eines Ringes vorliegt und daß der dem Beutelträger (10) zugeordnete Mechanismus so aufgebaut ist, um eines der Enden (45, 45') dieses Organs (24) dem anderen Ende, zu dem es zunächst unter Abstand stand, anzunähern.

5. Einrichtung nach Anspruch 3 oder Anspruch 4, dadurch gekennzeichnet, daß der Betätigungsmechanismus (80) des Spannorgans (24') zur Annäherung der zunächst unter Abstand voneinander stehenden Enden aneinander vom Kniehebeltyp ist.

6. Einrichtung nach Anspruch 3 oder Anspruch 4, dadurch gekennzeichnet, daß der Betägigungsmechanismus des Spannorgans vom Typ mit Exzenter oder dergleichen ist.

7. Einrichtung nach Anspruch 3 oder 4, dadurch gekennzeichnet, daß die Verformung des Spannorgans (24) in Abhängigkeit von der Betätigung des Mechanismus (48) durch Eingriff von Zähnen (46) an einem Ende (45) des Spannorgans (24) und von Zähnen (47) konjugierter Form ist, die auf einem Hebel (48) vorgesehen sind, der verschwenkbar auf diesem Bauteil (38) gelagert ist.

8. Einrichtung nach Anspruch 7, dadurch gekennzeichnet, daß dieser Hebel (48) verschwenkbar um eine Achse gelagert ist, die durch zwei Zapfen (49, 50), die sie trägt, materialisiert ist, und die in Augen (31, 33) konjugierter Form zweier Backen (30, 32) greifen, die radial auf diesem Teil (38) vorstehen.

9. Einrichtung nach einem beliebigen der Ansprüche 3 bis 9, dadurch gekennzeichnet, daß der Betätigungsmechanismus Verriegelungsmittel (51, 51'; 52, 53, 34) umfaßt.

10. Einrichtung nach einem der vorhergehenden Ansprüche, dadurch gekennzeichnet, daß der Ansatz (16) steifer als die Beutelnase (62) derart ist, daß die Innenfläche der Beutelnase mit der Außenfläche des Ansatzes untere Bildung einer linearen Kontaktzone zusammenwirkt, derart, daß die Dichtheit der Einrichtung unter der Wirkung des Spannorgans (24) sichergestellt ist, daß durch einen Effekt konjugierter Formen wirkt.

11. Einrichtung nach einem der vorhergehenden Ansprüche, dadurch gekennzeichnet, daß die Beutelnase (62) aus einem elastisch verformbaren und nachgiebigen Material besteht und einen geraden Querschnitt hat, der konjugiert zu dem des Spannorgans (24) einerseits ist und mit dem des Ansatzes (16) des Trägerbeutels (10) andererseits zusammenpaßt, um die dichte Verbindung des Beutels des Beutelträgers zu liefern.

12. Einrichtung nach einem der vorhergehenden Ansprüche, dadurch gekennzeichnet, daß die Dichtheit der Übergangsverbindung der Beutelnase (62) und des Beutelträgers (10) realisiert ist durch wenigstens einen Kontakt vom linearen Typ (71) zwischen der Außenfläche des Ansatzes (16) des Beutelträgers (10) und der Innenfläche (66) der Beutelnase (62).

13. Einrichtung nach einem der vorhergehenden Ansprüche, dadurch gekennzeichnet, daß die Oberfläche des Ansatzes (16) mit der die Nase des Beutels (62) zusammenwirkt, konisch und die (66) der Beutelnase gewölbt oder umgekehrt ist.

14. Einrichtung nach einem der Ansprüche 3 bis 13, dadurch gekennzeichnet, daß der gerade Querschnitt der Beutelnase (62) und der des Spannorgans (24) derart konjugiert sind, daß die Betätigung des Mechanismus (48), der die Annäherung der Enden des Spannorgans (24) sicherstellt, solange unmöglich ist, wie die Beutelnase (62) nicht korrekt bezüglich dieses Spannorgans und bezüglich des Ansatzes (16) des Beutelträgers (10) positioniert ist, während für eine quasi zufriedenstellende Positionierung der Beginn der Betätigung dieses Mechanismus durch einen Führungseffekt das vollkommene Einsetzen der die Einrichtung bildenden Teile bezüglich einander bis zum Blockieren durch den Effekt konjugierter Formen dieser Teile untereinander mit sich bringt.

15. Einrichtung nach einem der vorhergehenden Ansprüche, dadurch gekennzeichnet, daß die Beutelnase (62) vorzugsweise durch (Preßformen plastischen Materials, bevorzugt auf der Basis eines Copolymers von Ethylen und Vinylacetat (EVA) oder aus einem kompressiblen Polymer, wie einem Polyurethanschaum oder noch und bevorzugt aus einem zellenförmigen Material mit integrierter Oberflächenhaut auf der Basis von Polyethylen oder einem Mischpolymerisat aus Ethylen und Vinylacetat oder Polyurethan oder einem analogen Material gestaltet ist.

16. Einrichtung nach einem der vorhergehenden Ansprüche, dadurch gekennzeichnet, daß das Spannorgan (24) vorzugsweise aus einem plastischen steifen durch (Preß)formen in seinem "offenen" Zustand gestalteten Material besteht, um diesen Zustand nach dem Spannen wieder einzunehmen und besteht bevorzugt aus Polypropylen und/oder Polyamid oder aus ABS-Mischpolymerisat oder aus Polyethylen hoher Dichte.

17. Einrichtung nach Anspruch 15, dadurch gekennzeichnet, daß die Nase (62) der Tasche bevorzugt auf der Basis eines EVA-Mischpolymerisats oder eines Polyurethanelastomers oder noch, und bevorzugt, aus einem zellenförmigen Material mit integrierter Oberflächenhaut auf der Basis von EVA-Mischpolymerisat oder einem Elastomer von Polyurethan oder einem analogen Material vorliegt.

18. Einrichtung nach einem der vorhergehenden Ansprüche, dadurch gekennzeichnet, daß der Ansatz (16) an seinem vom Klebeschuh entfernten Ende und im Inneren seiner Öffnung entsprechend einem Randteil (16') geformt ist, das die Montage und das Halten eines Rings oder einer Schale einer Konvexität ermöglicht, die besonders nützlich in Geräten gewisser Invaginationsostomien ist.
